# EUROPEAN PATENT SPECIFICATION

(11) **EP 0 440 643 B1**
(45) Date of publication and mention of the grant of the patent: **03.09.1997**
(21) Application number: 89907213.6
(22) Date of filing: 09.06.1989
(51) Int. Cl.: C07C 255/54, C09K 19/12, C07C 255/56

(54) **FLUORINATED 4''-CYANO SUBSTITUTED TERPHENYLS**
FLUORINIERTE 4"-CYANOSUBSTITUIERTE TERPHENYLE
TERPHENYLS FLUORES 4''-CYANO SUBSTITUES

(30) Priority: 16.06.1988 GB 8814327
(43) Date of publication of application: 14.08.1991
(73) Proprietor: Secretary of State for Defence in Her Britannic Majesty's Gov. of the United Kingdom of Great Britain and Northern Ireland, London SW1A 2HB (GB)
(72) Inventor: COATES, David, Merley,Wimbourne BH21 3SW (GB); SAGE, Ian, Broadstone,Dorset BH8 EG (GB); GREENFIELD, Simon, Poole,Dorset BH17 7YA (GB); GRAY, George, William, Cottingham,North Humberside HU16 4DZ (GB); LACEY, David, Hull,North Humberside HU18 9NN (GB); TOYNE, Kenneth, Johnson, Hull,North Humberside HU6 8QW (GB); CHAN, Lawrence, Kam, Ming, Northholt,Middlesex UB5 5UQ (GB); HIRD, Michael, Hull,North Humberside HU4 7B2 (GB)
(74) Representative: Beckham, Robert William
(86) International application number: GB8900647
(87) International publication number: WO8912621

(56) References cited:
- EP-A- 0 132 377
- WO-A-86/04081
- WO-A-86/04324
- DE-A- 2 356 085
- DE-A- 2 639 838
- DE-A- 2 939 782
- GB-A- 2 039 937
- Molecular Crystals and Liquid Crystals, vol. 158B, May 1988 Gordon & Breach Science Publishers S.A., US, L.K.M. Chan et al.: "Synthesis and liquid crystal behaviour of further 4,4"-disubstituted 2'-fluoro-1,1':4'1"-terphenyls", pages 209-240
- Molecular Crystals and Liquid Crystals + Letters, vol. 123, nos. 1/4, 1985, Gordon & Breach Science Publ. Inc., New York, US; L.K.M. Chan et al.: "Synthesis and evaluation of some 4,4"-disubstituted lateral fluoro-1,1':4'1"- terphenyls", pages 185-204
- Molecular Crystals and Liquid Crystals, vol. 38, nos. 1-4, 1977, Gordon & Breach Science Publishers, Ltd, New York, US; B.K. Sadashiva et al.: "A convenient method for the preparation of 4-n-alkyl-4"-cyano-p-terphenyl", pages 345-252

## Description

This invention relates to novel fluorinated 4''-cyano substituted terphenyls and to liquid crystal materials which incorporate them, in particular liquid crystal materials which show a nematic phase. Such liquid crystal materials may be used industrially as the working fluid of electro-optic display devices.

The use in liquid crystal materials of 4-alkyl and 4-alkoxy-4"-cyano terphenyls is described in GB 1433130, among many other publications and liquid crystal materials containing them are widely used. GB1433130 suggests the possibility of one of the three phenyl rings being laterally substituted, suggested substituents being the bulky methyl group or an unspecified halogen. No example of any such laterally substituted terphenyl is disclosed in GB1433130, no method for their preparation is given, and no preference for any particular halogen substituent is expressed.

GB2039937A describes liquid crystal materials which contain at least one laterally substituted 4-alkyl-4"-cyanoterphenyl of general structure: 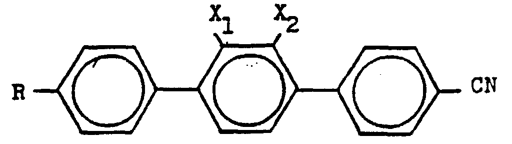 where R is alkyl, one of X₁ or X₂ being hydrogen and the other being methyl or a halogen preferably the bulky chlorine. A complex 6 step method for preparation of these terphenyls is described in GB2039937A, but no example of a terphenyl having X₁, or X₂ as a halogen is described, the implication being that none were made.

Mol. Cryst. Liq. Cryst. (1985) 123 169-177 describes 2 and 2' fluorosubstituted 4-alkyl-4'-cyanobiphenyls, but the data presented therein suggests that lateral fluorination increases the melting point of these compounds, and that their solubility in liquid crystal materials is low.

Laterally fluoro-substituted terphenyls of general structure: 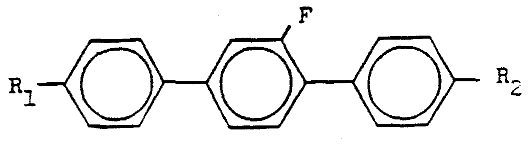 where R₁ and R₂ are alkyl or alkoxy are known and examples are given in EP-A-0132377. The preparation method described therein cannot easily be applied to the preparation of a terminally cyano substituted terphenyl. Mol. Cryst. Liq. Cryst., 1988, vol. 58B, pp209-40 discloses compounds of the formula: 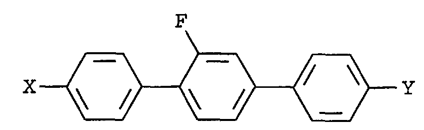 wherein X may be inter alia alkyl or alkoxy and Y may be inter alia CN.

The present invention has identified a class of terphenyls which on the basis of the prior art discussed above appear to be newly prepared and which show advantageous properties.

According to the present invention, fluorinated 4"-cyano substituted terphenyls are provided, of general formula I: 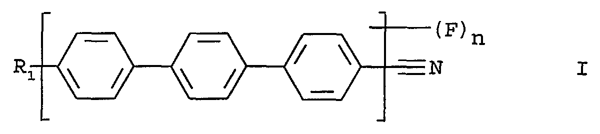 wherein R₁ is selected from alkenyl, R, RO, RCO and hydrogen, where R is alkyl or perfluoroalkyl containing up to 12 carbon atoms, n is 2, the fluoro substituents being in any of the (2,3"), (3,3"), (3, 2"), (2,2"), (3,3'), (3, 2') (2,2'), (2,3'), (3', 2"), (3',3"), (2', 2"), (3,5), (2,6), (2,5), (3'5'), (2', 6'), (2',5'), (3", 5"), (2", 6"), (2", 5"), (2,3), (2',3'), (2", 3") or (2', 3") pairs of positions on the terphenyl.

The structural preferences expressed below are inter alia on the basis of usefulness or constituents of liquid crystal mixtures and ease of preparation.

The group R₁ is preferably alkyl or alkoxy and may be straight chain (n), branched (iso) or may contain an asymmetrically substituted carbon atom so as to be a chiral group. When R₁ is organic it preferably contains from 2 to 9 carbon atoms, especially from 2 to 7. Particularly useful compounds are those in which R₁ is n-alkyl or n-alkoxy, or the chiral groups (+) or (-) 2-methylbutyl, 2-methylbutyloxy, 3-methylpentyl, 3-methylpentyloxy, 4-methylhexyl or 4-methylhexyloxy.

The nomenclature: 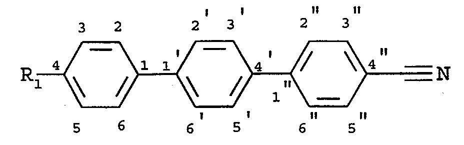 is used for the lateral substitution positions. 2', 3"-difluoro, 2, 3"-difluoro and 3", 5"-difluoro substituted compounds of formula I are particularly preferred.

According to a further aspect of this invention a liquid crystal composition is provided containing at least two components, characterised in that at least one is a fluorinated 4"-cyano substituted terphenyl of general formula I: 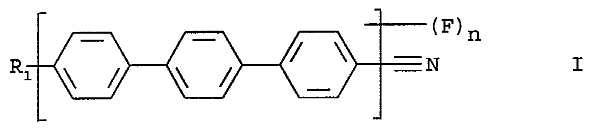 wherein R₁ is selected from alkenyl, R, RO, RCO or hydrogen, where R is alkyl or perfluoroalkyl containing up to 12 carbon atoms, n is 1 or 2 or 3 and the fluorosubstituent(s) may be in any of the available substitution positions.

A suitable method for the preparation of a compound of formula I as defined above is one in which a boronic acid of formula II: 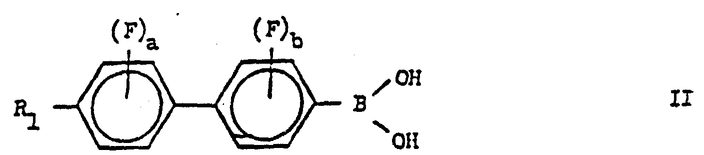 wherein R₁ is as defined in formula I, a and b are 0,1,2 or 3 is coupled with a benzonitrile of formula III: 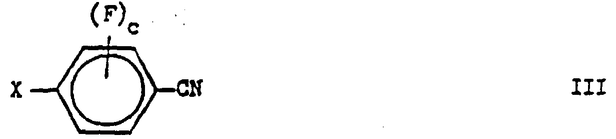 wherein X is chlorine, bromine or iodine, c is 0,1,2 or 3, provided (a+b+c) is 1,2 or 3, to form the compound of formula I.

In this method coupling occurs between the ring positions to which the B(OH)₂ and X groups are attached. The indicated fluorine substituent(s) (F) may be in any of the available substitution positions. Preferably (a+b+c) is 1 or 2 and there are one or two fluorine substituents in the compounds of formulae II and III which are preferably disposed so as to form compounds of formula I as listed in table I, and suitable fluoro substitution patterns for compounds of formulae II and III will be apparent to those skilled in the art.

Boronic acids II may be prepared starting from known biphenyls IV: 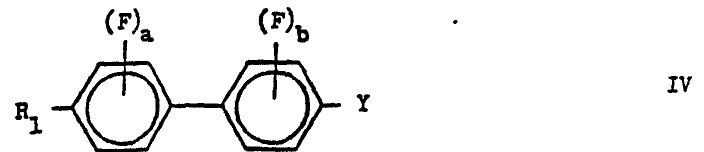 wherein Y is chlorine, bromine or iodine, preferably bromine. Biphenyls IV in which R₁ is hydrogen may be converted into those in which R₁ is R or RCO by means of a Friedel-Crafts reaction using an acyl chloride RCOC1 to add a group RCO at the available 4-position, which may then be reduced to an alkyl group using for example hydrazine hydrate, or a trialkyl silane, triethyl silane being preferred.

To prepare the boronic acid II, the biphenyl IV is preferably first converted into a Grignard reagent by reaction of the biphenyl IV with magnesium metal in for example an ether such as tetrahydrofuran (THF). Conditions for preparation of Grignard reagents are well known.

The Grignard reagent may then be reacted with a trialkyl borate (R'O)₃B, where R' is alkyl, preferably methyl or isopropyl, conveniently in the same solvent in which the Grignard reagent was prepared. The resulting boronated product is then hydrolysed, for example with a mineral acid, preferably hydrochloric acid. The compound II may then be isolated and purified conventionally. Some methods for preparation of unfluorinated, monofluorinated and difluorinated biphenyls of formula IV are described in detail in WO 89/02425 and PCT/GB 88/00880.

Benzonitriles of formula III are known and are available commercially or may be synthesised, eg as described in GB 8804330 A or PCT/GB 89/00178 Preferably X is bromine. The coupling step is preferably carried out in the presence of a coupling agent, such as a palladium (0) catalyst, preferably tetrakis (triphenylphosphine) palladium (0). Conveniently an alcohol solvent may be used for the coupling reaction. The product compound of formula I may then be isolated and purified by conventional methods.

A third aspect of this invention is a liquid crystalline material, containing at least two components, at least one of which is a compound of formula I. This liquid crystalline material is preferably a nematic or cholesteric liquid crystalline material. Such materials may be used on electro-optic display devices such as watches and calculators etc, and also in thermochromic displays in which the colour or reflected light varies with temperature.

Compounds of formula I, in particular the preferred compounds referred to above, have a number of desirable properties which make them very useful components of liquid crystal materials. They often have higher nematic to isotropic (N-I) clearing point temperatures and lower melting points (C-N) than their unfluorinated counterparts. They generally have a high birefringence. They also generally show a higher solubility in commonly used liquid cystal materials than their unfluorinated counterparts. These advantages are not suggested by the prior art referred to above.

Suitable compounds for the other components of the liquid crystal material will be apparent to those skilled in the field, and will depend upon the properties such as dielectric anisotropy, birefringence, working temperature range etc required in the material for the application for which the material is intended. Some types of suitable material are discussed briefly below.

Preferably as well as containing one or more formula I compounds the mixture contains one or more compounds of formula V: 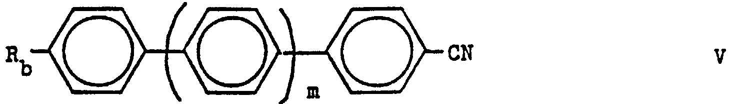 wherein R_{b} is alkyl or alkoxy, preferably containing 1 to 8 carbon atoms, and preferably straight chain, and wherein m is 0 or 1. Such compounds are included in the subject matter of GB 1433130.

Compounds of formula I have a positive dielectric anistropy , and the material may for example contain other liquid crystalline compounds which have a positive dielectric anistropy , for example as described in EP-A-0132377, particularly in Fig 8 thereof and the related text.

The material may alternatively or also contain liquid crystalline compounds of low dielectric anistropy, for example to form a mixture of intermediate dielectric anistropy , or a thermochromic mixture. Some examples of such compounds are described in EP-A-0132377, particularly in Fig 9 thereof and the related text.

The material may alternately or also contain liquid crystalline compounds having a high clearing point, for example to raise the N-I transition temperature. Some examples of such compounds are described in EP-A-0132377, particularly in Fig 10 thereof and the related text.

To cause the material of this aspect of the invention to show a cholesteric (Ch) (or chiral nematic) phase the material must contain at least one compound containing an asymmetric carbon atom. This may be a chiral compound of formula I, or alternatively or also the material may for example contain one or more chiral compound of formula V above, eg (+) or (-) 4-(2-methylbutyl)-4'-cyano biphenyl or 4-(2-methylbutyloxy)-4'-cyano biphenyl.

The material may also contain one or more pleochroic dyes, for example the dyes described in EP-A-82300891.7.

The proportions of these components used in the material of this aspect of the invention trill depend upon the intended application, and the material may usefully contain two or more compounds of formula I having different substituents or different dispositions of subsituents on the terphenyl system.

The materials of this aspect of the invention may be used in any of the known forms of liquid crystal display device, for example a twisted nematic effect device, Freedericksz effect device, cholesteric memory mode device, cholesteric to nematic phase change effect device, dynamic scattering effect device, two frequency switching effect device, a "supertwist" effect device, or a thermometer using a thermochromic material. The method of construction and operation of such devices, and characteristics of a liquid crystal material suitable for use therein, are well known in the field. A liquid crystal display device which incorporates as its working fluid a material as described above, constitutes a fourth aspect of this invention.

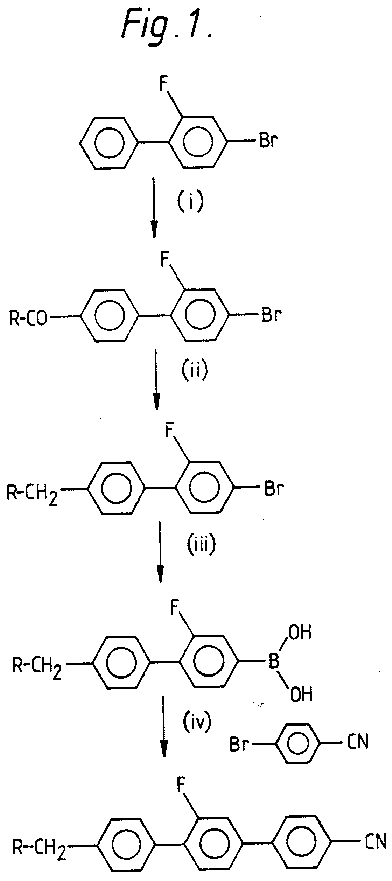

The invention will now be described by way of example only with reference to Fig 1 which shows the preparative route used in example 1 starting from 2-fluoro-4-bromo biphenyl to prepare a 4-alkyl-2'-fluoro-4"-cyano terphenyl, R in Fig 1 being alkyl.

In these examples: C-N = solid crystal to nematic liquid crystal transition, N-I = nematic liquid crystal to isotropic liquid transition, S_{A} = smectic A liquid crystal phase. E7, E8 and K15 are Trade Marks of commercially available liquid crystal materials from BDH Ltd., Poole, GB and ZLI 1132 is a Trade Mark of a commercially available liquid crystal material from E. Merck GmbH, Darmstadt DE. The compositions of these materials have been published.

### Example 1

Preparation of compounds of general structure: 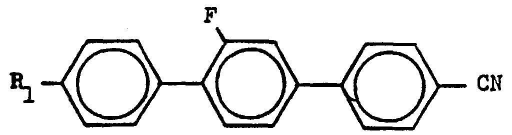 where R₁ is n-alkyl.

### Step (i) Synthesis of ethyl 4-(4'-bromo-2'-fluorobiphenyl) ketone

To a suspension of aluminium chloride (40g;0.3mol) in dichloromethane(100ml) was slowly added, with stirring and ice bath cooling, a solution of propionyl chloride (20.9g;0.29mol) and 2-fluoro-4-bromobiphenyl(62.5g;0.25mol) in dichloromethane (10ml). The mixture was stirred for 16 hrs at 20°C and then carefully poured into ice/water (500g), the organic material was extracted into dichloromethane (2x200ml), washed with brine (2x100ml) and dried over sodium sulphate. After evaporation of the dichloromethane, the solid was crystallised from two volumes of industrial spirits. Yield 62.3g; 70%.

### Step (ii) Synthesis of 4-bromo-2-fluoro-4'-propylbipheyl

The product from step (i) 60g;0.196 mol) was dissolved in trifluoroacetic acid (100ml) and triethyl silane (76ml; .476mol) was added dropwise while the temperature of the mixture was maintained at 10-20°C and then stirred for 16hrs at 20°C. The reaction mixture was then poured into water (1L) and extracted with ether (3x100ml). After drying and evaporation of the ether, the product was distilled under high vacuum (1.5mmHg). Yield 51.4g;90%.

### Step (iii) Synthesis of 4'propyl-2-fluoro-4-biphenylboronic acid

The product from step (ii) (10g;0.034mol) in tetrahydrofuran (35ml) was added dropwise to a suspension of magnesium turnings (0.88g) in tetrahydrofuran (5ml). The mixture was warmed to 75°C when the Grignard reaction started. Further of the bromo ccmpound was slowly added and the external heat source removed, a gentle reflux being maintained. After complete addition, the mixture was heated for 30mins under reflux and then cooled to 15°C.
A dry nitrogen atmosphere was introduced and trimethyl borate (3.77g; 0.0363mol) was slowly added and the mixture stirred at 15-20°C for 16hrs. A 20% soln of hydrochloric acid (50ml) was slowly added, the organic layer was separated, and the aqueous layer extracted with ether (2x50ml). The combined organic layers were washed with brine and dried over sodium sulphate. Upon evaporation of the solvents a yellow solid was obtained. Yield 8.7g; 100%.

### Step (iv) Synthesis of 4-propyl-2'-fluoro-4"-cyano 1,1': 4',1" terphenyl

The crude product from step (iii) (8.7g;0.034mol) 4-bromobenzonitrile (5.0g;0.0275mol), tetrakis(triphenylphosphine) palladium (0.64g) and methylated spirits (20ml) were heated under reflux with vigorous stirring for 5hrs. After cooling, the organic layer was separated, washed with water, dried over sodium sulphate and evaporated to dryness. The crude product was purified by column chromatography on silica gel followed by crystallisation from ethyl acetate. Yield 5.3g;51%.

The properties of some compounds prepared by the method of example 1, using an appropriate acyl chloride in step (i) are listed below:

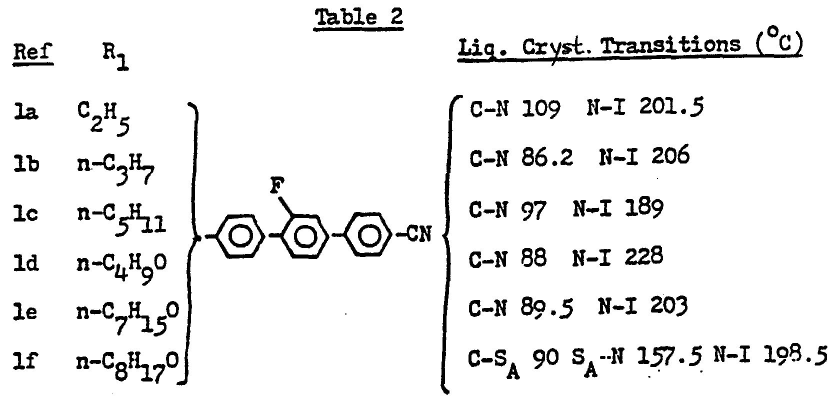

### Example 2

Other compounds of formula I were prepared using a method analogous to that of example 1, as listed below:

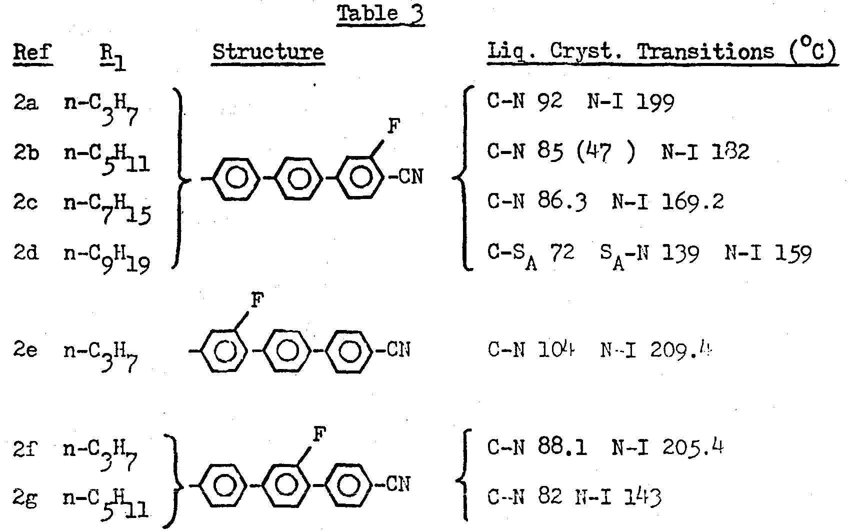

### Example 3

The following difluorinated terphenyls were prepared using the method of example 1 and appropriately fluorinated starting materials;

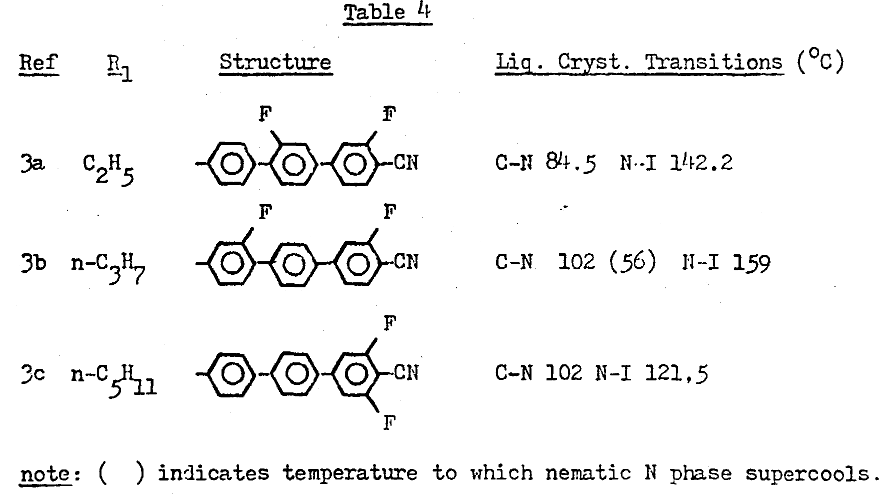

Some comparative data for unfluorinated analogues of these compounds is listed below:

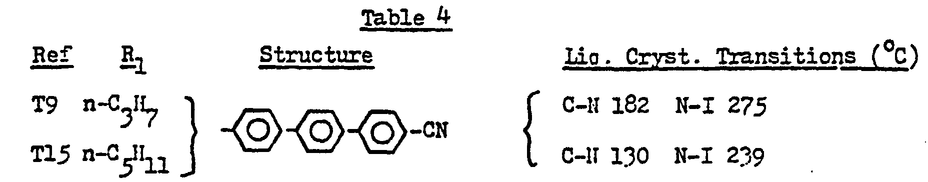

From this data it can be seen that the melting point of the fluorinated compounds of the invention is substantially lower than those of the unfluorinated analogues, with little change in the breadth of the nematic phase.

### Example 4

Some data relating to liquid crystalline mixtures containing compounds of the invention is presented in tables 5 and 6 below. These compositions contain biphenyls and terphenyls of formula V, E7 having the composition:

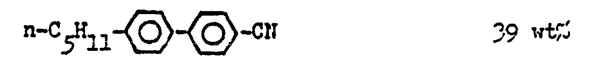

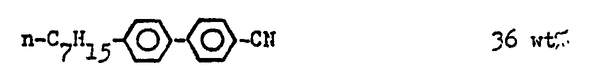

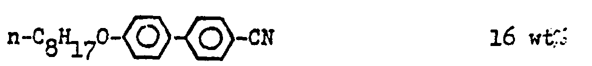

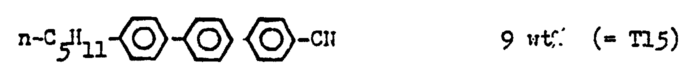

From tables 5 and 6 it can be seen that the fluorinated compounds of the invention have an improved birefringence and solubility in liquid crystals containing them relative to their unfluorinated analogues and that their incorporation in liquid crystal materials such as E8 can improve the N-I transition temperature and birefringence.

It was found to be difficult to freeze a 30 wt% solution of 1b in E7, and this factor allows the N-I and Δ n of mixtures to be increased.

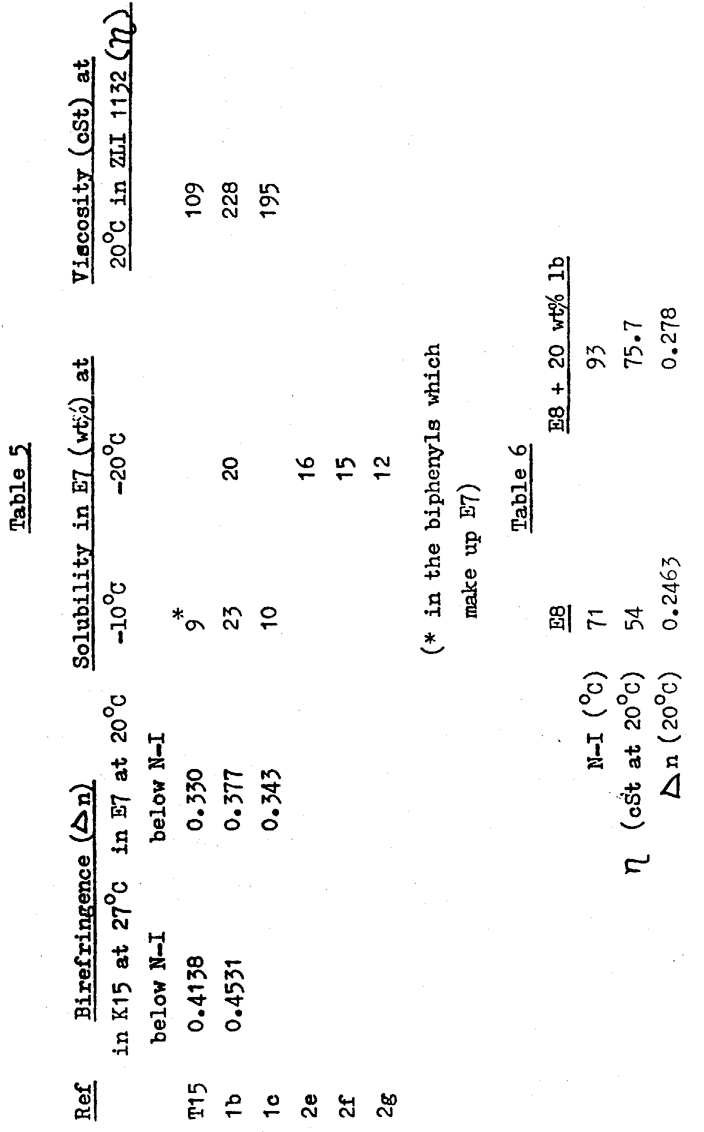

## Claims

1. Fluorinated 4"-cyano substituted terphenyl of general formula I: 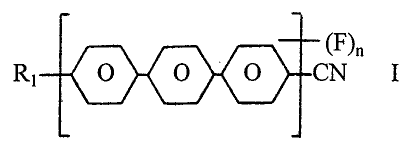 wherein R₁ is selected from alkenyl, R, RO, RCO and hydrogen, where R is alkyl or perfluoroalkyl containing up to 12 carbon atoms, n is 2, the fluoro substituents being in any of the (2,3"), (3,3"), (3, 2"), (2,2"), (3,3'), (3, 2') (2,2'), (2,3'), (3', 2"), (3',3"), (2', 2"), (3,5), (2,6), (2,5), (3'5'), (2', 6'), (2',5'), (3", 5"), (2", 6"), (2", 5"), (2,3), (2',3'), (2", 3") or (2', 3") pairs of positions on the terphenyl.

2. Terphenyl according to claim 1 characterised in that R₁ is C₂-C₉ alkyl or alkoxy.

3. Terphenyl according to claim 2 characterised by being a 4-alkyl or alkoxy 2', 3"-difluoro-4"-cyano terphenyl.

4. Terphenyl according to claim 2 characterised by being a 4-alkyl or alkoxy 2, 3"-difluoro-4"-cyano terphenyl.

5. Terphenyl according to claim 2 characterised by being a 4-alkyl or alkoxy 3", 5"-difluoro-4"-cyano terphenyl.

6. Liquid crystal composition containing at least two components, characterised in that at least one is a fluorinated 4"-cyano substituted terphenyl of general formula I 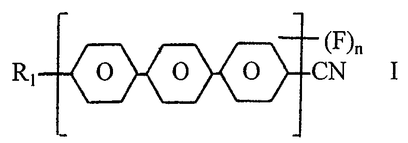 wherein R₁ is selected from alkenyl, R, RO, RCO or hydrogen, where R is alkyl or perfluoroalkyl containing up to 12 carbon atoms, n is 1 or 2 or 3 and the fluorosubstituent(s) may be in any of the available substitution positions.

7. A liquid crystal composition according to claim 6, characterised in that as well as containing at least one terphenyl of formula I the composition also contains at least one compound of formula V: 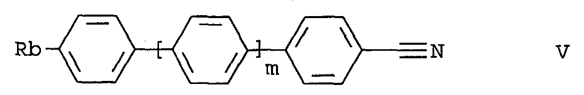 wherein Rb is C₁-C₈ alkyl or alkoxy and m is 0 or 1.

8. An electro-optic or thermochromic display device comprising a composition as claimed in claim 6 or 7.

## Patentansprüche

1. Fluoriertes 4"-cyanosubstituiertes Terphenyl der allgemeinen Formel (I): 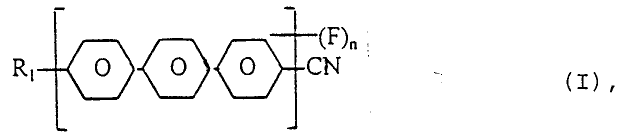 worin R₁ unter Alkenyl, R, RO, RCO und Wasserstoff ausgewählt ist, wobei R Alkyl oder Perfluoralkyl mit bis zu 12 Kohlenstoffatomen bedeutet, und n gleich 2 ist, wobei sich die Fluorsubstituenten in einer der Stellungen (2,3"), (3,3"), (3,2"), (2,2"), (3,3'), (3,2'), (2,2'), (2,3'), (3',2''), (3',3,"), (2',2''), (3,5), (2,6), (2,5), (3',5'), (2',6'), (2',5'), (3",5"), (2'',6''), (2",5''), (2,3), (2',3'), (2'',3'') oder (2',3'') des Terphenyls befinden.

2. Terphenyl nach Anspruch 1, dadurch gekennzeichnet, daß R₁ C₂₋₉-Alkyl oder C₂₋₉-Alkoxy bedeutet.

3. Terphenyl nach Anspruch 2, dadurch gekennzeichnet, daß es ein 4-Alkyl-2",3"-difluor-4"-cyanoterphenyl oder 4-Alkoxy-2",3"-difluor-4"-cyanoterphenyl ist.

4. Terphenyl nach Anspruch 2, dadurch gekennzeichnet, daß es ein 4-Alkyl-2',3''-difluor-4"-cyanoterphenyl oder 4-Alkoxy-2,3''-difluor-4"-cyanoterphenyl ist.

5. Terphenyl nach Anspruch 2, dadurch gekennzeichnet, daß es ein 4-Alkyl-2",3"-difluor-4"-cyanoterphenyl oder 4-Alkoxy-3",5"-difluor-4"-cyano-terphenyl ist.

6. Flüssigkristallzusammensetzung, die mindestens zwei Komponenten enthält, dadurch gekennzeichnet, daß mindestens eine Komponente ein fluoriertes 4"-cyanosubstituiertes Terphenyl der allgemeinen Formel (I) 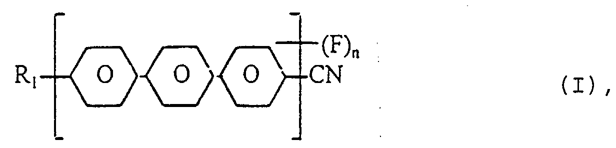 ist, worin R₁ unter Alkenyl, R, RO, RCO oder Wasserstoff ausgewählt ist, wobei R Alkyl oder Perfluoralkyl mit bis zu 12 Kohlenstoffatomen bedeutet, n 1 oder 2 oder 3 bedeutet, und sich der oder die Fluorsubstituent(en) in beliebigen Substitutionsstellungen befinden können.

7. Flüssigkristallzusammensetzung nach Anspruch 6, dadurch gekennzeichnet, daß die Zusammensetzung neben mindestens einem Terphenyl der Formel (I) auch mindestens eine Verbindung der Formel (V) 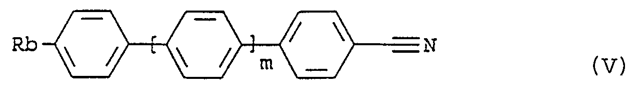 enthält, worin Rb C₁₋₈-Alkyl oder C₁₋₈-Alkoxy bedeutet und m Null oder 1 ist.

8. Elektrooptische oder thermochrome Anzeigevorrichtung, die eine Zusammensetzung nach Anspruch 6 oder 7 umfaßt.

## Revendications

1. Terphényle fluoré 4"-cyano-substitué de formule générale I : 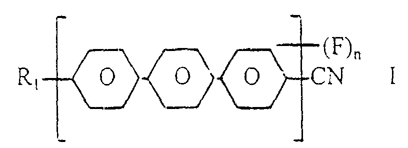 dans laquelle R₁ est choisi entre des groupes alcényle, R, RO, RCO et l'hydrogène, R étant un groupe alkyle ou perfluoralkyle contenant jusqu'à 12 atomes de carbone, n est égal à 2, les substituants fluoro occupant l'une quelconque des paires de positions (2, 3'') , (3, 3'') , (3,2"), (2,2"), (3,3'), (3,2'), (2,2'), (2,3',), (3',2"), (3',3"), (2',2"), (3,5), (2,6), (2,5), (3',5'), (2', 6') , (2',5'), (3'', 5''), (2'',6"), (2",5"), (2,3), (2',3'), (2",3") ou (2',3") sur le terphényle.

2. Terphényle suivant la revendication 1, caractérisé en ce que R₁ est un groupe alkyle ou alkoxy en C₂ à C₉.

3. Terphényle suivant la revendication 2, caractérisé en ce qu'il est un 4-alkyl- ou -alkoxy 2',3'-difluoro-4"-cyano-terphényle.

4. Terphényle suivant la revendication 2, caractérisé en ce qu'il est un 4-alkyl- ou -alkoxy 2,3"-difluoro-4"-cyano-terphényle.

5. Terphényle suivant la revendication 2, caractérisé en ce qu'il est un 4-alkyl- ou -alkoxy 3",5"-difluoro-4"-cyano-terphényle.

6. Composition à cristaux liquides contenant au moins deux composants, caractérisée en ce que l'un au moins est un terphényle fluoré 4"-cyano-substitué de formule générale I 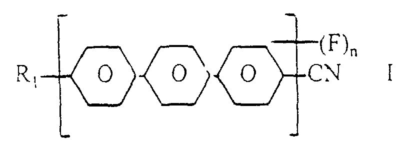 dans laquelle R₁ est choisi entre des groupes alcényle, R, RO, RCO ou l'hydrogène, R représentant un groupe alkyle ou perfluoralkyle contenant jusqu'à 12 atomes de carbone, n a la valeur 1 ou 2 ou 3 et le ou les substituants fluoro peuvent occuper l'une quelconque des positions disponibles de substitution.

7. Composition à cristaux liquides suivant la revendication 6, caractérisée en ce que, de même qu'elle contient au moins un terphényle de formule I, elle contient aussi au moins un composé de formule V 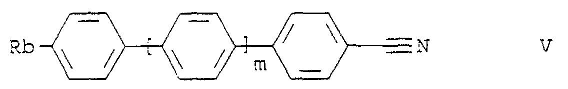 dans laquelle R_{b} est un groupe alkyle ou alkoxy en C₁ à C₈ et m a la valeur 0 ou 1.

8. Dispositif à affichage électro-optique ou thermochrome, comprenant une composition selon la revendication 6 ou 7.
